# EUROPEAN PATENT SPECIFICATION

(11) **EP 2 833 147 B1**
(45) Date of publication and mention of the grant of the patent: **13.09.2017**
(21) Application number: 13768453.6
(22) Date of filing: 29.03.2013
(51) Int. Cl.: G01N 33/569, C12Q 1/70, G01N 33/543

(54) **DETECTION KIT FOR INFLUENZA A VIRUS**
KIT FÜR DEN NACHWEIS VON INFLUENZA-A-VIRUS
KIT DE DÉTECTION DU VIRUS DE LA GRIPPE A

(30) Priority: 30.03.2012 JP 2012081762
(43) Date of publication of application: 04.02.2015
(73) Proprietor: Tanaka Kikinzoku Kogyo K.K., Chiyoda-ku Tokyo 100-6422 (JP)
(72) Inventor: IWAMOTO, Hisahiko, Hiratsuka-shi Kanagawa 254-0076 (JP); KAWAMOTO, Hiroko, Hiratsuka-shi Kanagawa 254-0076 (JP); KATO, Shinichi, Hiratsuka-shi Kanagawa 254-0076 (JP)
(74) Representative: J A Kemp
(86) International application number: PCT/JP2013/002150
(87) International publication number: WO 2013/145767

(56) References cited:
- WO-A1-2005/007697
- JP-A- 2006 071 631
- JP-A- 2007 105 038
- JP-A- 2010 539 162
- JP-A- 2012 529 885
- US-A1- 2010 081 125
- US-A1- 2011 129 816
- Gui-Rong Bai ET AL: "FULL PAPER Virology Improvement of a Rapid Diagnosis Kit to Detect Either Influenza A or B Virus Infections", J. Vet. Med. Sci, 1 January 2006 (2006-01-01), pages 35-40, XP55221418, Retrieved from the Internet: URL:https://www.jstage.jst.go.jp/article/j vms/68/1/68_1_35/_pdf
- "X/Pect FLU A&B", , 28 August 2003 (2003-08-28), XP055221363, Retrieved from the Internet: URL:http://www.oxoid.com/pdf/ca/X-pect-Flu -A-and-B.pdf [retrieved on 2015-10-15]

## Description

### Technical Field

The present invention relates to a test kit by immunochromatography for detecting influenza A virus, using an antibody specifically causing an antigen-antibody reaction with an influenza A virus nuclear protein but not causing an antigen-antibody reaction by Western blotting with a full-length influenza A virus nuclear protein separated using SDS (sodium dodecyl sulfate)-polyacrylamide gel electrophoresis.

### Background Art

Influenza means an infectious disease caused by influenza virus. It is known that as the typical symptom, fever, headache, physical weariness, myalgia, arthralgia, and the like suddenly appear, and cough, nasal discharge, and the like follow in tandem, and it is said that these symptoms subside in around a week. As compared with other so-called cold syndromes, characteristics of influenza are the severe systemic symptoms. In order to make an accurate diagnosis, virological support is required. In the time when influenza is epidemic, it is important to make an accurate diagnosis for the patient with a cold symptom whether the patient has influenza or not, not only in view of the appropriate selection of an anti-influenza virus agent for the treatment, but also in view of the epidemiology of the precise grasp of epidemic state and the determination of the effect of influenza vaccine.

In order to make an accurate pathogen diagnosis of influenza, there is a standard technique of virus separation using a pharyngeal swab or a gargle liquid as the material, however, it takes a long time to obtain the diagnosis. If the viral genome is detected by using polymerase chain reaction (PCR), a highly precise result can be obtained in a short period of time. However, a PCR method requires a special apparatus, and thus can be performed only in an institute for health or a limited laboratory.

In recent years, a rapid diagnostic kit with which an influenza antigen can be detected at a bed side, in an outpatient care clinic, or the like becomes available on the market, and thus virological diagnosis has become performed easily in a daily clinical practice. A rapid diagnostic kit for influenza (see Patent Literatures 1 to 11), to which a principle of immunochromatography is applied, can obtain a test result by a simple operation in a short period of time using a biological sample such as nasal mucosa or pharyngeal mucosa that can be easily collected, and thus has the advantage of being less burden for both of the patient who is subjected to inspection and the health care worker who carries out the inspection. In addition, in the case where the result of "positive" is obtained, useful information can be provided for a doctor to make an accurate diagnosis. However, a rapid diagnostic kit currently available on the market does not have sufficient detection sensitivity of influenza virus, and thus even if the result of "negative" is obtained, the viral infection cannot be necessarily denied.

It is known that the amount of the influenza virus detected from nasal mucosa or pharyngeal mucosa of an influenza patient reaches the peak 2 to 3 days after the onset, then decreases rapidly, and disappears in 5 to 7 days. In order to determine as "positive" by a rapid diagnostic kit, it is necessary that the influenza virus proliferates in a living body after the infection and the amount of virus in a biological sample reaches an amount with which the detection sensitivity is available or more. There is a problem of determination of false negative that in a case of a patient in an initial stage of infection that is soon after the start of virus proliferation, a patient in which the proliferation rate of virus is suppressed due to the inoculation of vaccine, or the like, there may be a case where the sufficient amount of virus is not present in the biological sample to determine as positive, and thus the result of the rapid diagnostic kit becomes "negative" although the patient is actually infected with influenza virus. In a clinical practice, a test by a rapid diagnostic kit can stably detect the virus if 24 hours elapses after the onset and a highly precise result can be obtained, however, there may be a case where virus cannot be detected and the accurate determination cannot be obtained within 12 hours after the onset. From the situation described above, re-inspection is carried out for the patient with a result of "negative" on or after the next day depending on the other findings, and the patient has to seek medical attention again, this situation has forced the excessive burden in terms of cost and time.

In addition, also in view of the selection of an anti-influenza virus agent for treatment, the accurate diagnosis of influenza is required at the earliest possible time of the infection. At the moment, as a representative example of the therapeutic agent for influenza, a neuraminidase inhibitor such as oseltamivir phosphate (trade name: Tamiflu) and zanamivir (trade name: Relenza) is widely used. Neuraminidase plays an important role when influenza virus infects cell and propagates from cell to cell in a living body. A neuraminidase inhibitor inhibits the activity of neuraminidase, thus inhibits the influenza virus proliferated in a cell from exiting outside the cell, and exerts a therapeutic effect by suppressing the propagation of virus between cells. It is considered that an anti-influenza virus agent composed of such a neuraminidase inhibitor is effective if the agent is taken as soon as possible after the onset. It is considered that the agent is ideally taken within 12 hours after the onset, although the sufficient effectiveness can be obtained if the agent is taken within 24 hours after the onset, and the therapeutic effect becomes poor if the agent is taken more than 48 hours after the onset. However, while the treatment with an anti-influenza virus agent is required to be started at an early stage, the side effect of neuraminidase inhibitor is known, in addition, there exists a social request that an anti-influenza virus agent should be appropriately used in preparation for the explosive epidemic of influenza, accordingly the anti-influenza virus agent is required to be prescribed under the accurate diagnosis of influenza virus infection. Therefore, in a rapid diagnostic kit for influenza widely used in a clinical practice, the performance with which the detection sensitivity of influenza virus is improved and the determination of "positive" can be stably obtained with high accuracy at the earliest possible time after the onset and even if particularly within 24 hours after the onset is strongly required.

Patent Literature 12 relates to a device for detecting or quantifying influenza viruses in a sample, which comprises a detection region having a human anti-influenza virus nucleoprotein antibody immobilized onto a support, a sample supply region, and a sample-migrating region; and a kit for detecting or quantifying influenza viruses, which comprises a solid phase in which a human anti-influenza virus nucleoprotein antibody is fixed onto a carrier.

Non-Patent Literature 1 relates to a kit for rapid diagnosis of influenza by detecting influenza A or B virus specific nucleoproteins which involves using monoclonal antibodies FVA2-11 and FrB1-03 to recognise the epitope on the amino acid region 59-130aa of the nucleoprotein molecule of the influenza A virus and that on the region 72-191aa of the nucleoprotein of influenza B virus, respectively.

Patent Literature 13 describes immunoassays for detecting one or more target analytes in a fluid sample wherein the detection reaction occurs on a solid support and involves an amplification system. This document further describes making and using a test device having at least one site for detecting the presence of at least one target analyte, wherein a conjugate comprising dextran-polystreptavidin is immobilized at the test site (s) as a capture reagent for a complex containing the target analyze.

Non-Patent Literature 2 describes an *in vitro* immunochromatographic test for the direct, qualitative detection of influenza A and influenza B viral antigen from nasal wash, nasal swab and throat swab specimens from symptomatic patients. The test is intended as an aid in the rapid diagnosis of influenza A and influenza B viral infections.

### Citation List

### Patent Literature

Patent Literature 1: Japanese Patent Application Laid-Open (JP-A) No. 2011-069800
Patent Literature 2: JP-A No. 2010-261912
Patent Literature 3: JP-A No. 2007-093292
Patent Literature 4: JP-A No. 2007-033293
Patent Literature 5: JP-A No. 2006-194688
Patent Literature 6: JP-A No. 2006-194687
Patent Literature 7: JP-A No. 2006-189317
Patent Literature 8: JP-A No. 2006-067979
Patent Literature 9: International Publication WO 2009/148150
Patent Literature 10: International Publication WO 2005/007697
Patent Literature 11: International Publication WO 2005/007698
Patent Literature 12: US 2011/129816 A1
Patent Literature 13: US 2010/081125 A1

Non-Patent Literature 1: Giu-Rong Bai et al, J. Vet. Med. Sci., 68(1), 2006, 35-40
Non-Patent Literature 2: http://www.oxoid.com/pdf/ca/X-pect/Flu-A-and-B.pdf, 28 August 2003

### Technical Problem

An object of the present invention is to provide a test kit for influenza A virus that is a test kit for rapid diagnosis of influenza, to which a principle of immunochromatography is applied, and in which the detection sensitivity of influenza A virus is higher than that of a conventional test kit, and the determination of "positive" can be stably obtained with high accuracy at the earliest possible time after the onset of influenza.

The present inventors carried out intensive studies in order to enhance the detection sensitivity of a rapid diagnostic kit for influenza to which a principle of immunochromatography is applied, and to use an antibody having excellent affinity for an influenza A virus nuclear protein, for an antibody immobilized to a chromatography medium and an antibody conjugated with a labeling substance. In consideration that the test kit is used in a clinical practice, it cannot be said that there is no possibility of occurrence of the denaturation of an influenza A virus nuclear protein after the collection of biological sample for test, and there is a request that the characteristics of the antibody used for a test kit are clarified as much as possible, and thus the present inventors searched the antibody not only having excellent affinity for a native influenza A virus nuclear protein but also having similarly high affinity for an influenza A virus nuclear protein that is denatured, for example, separated using SDS-polyacrylamide gel electrophoresis (also referred to as SDS-PAGE), as an antibody used for a test kit, and tried to use the antibody for a test kit. In the antibody showing high affinity even for the influenza A virus nuclear protein separated using SDS-PAGE, there is the advantage that the binding ability can be less affected by the change of the structure of a nuclear protein, and the epitope region recognized by the antibody is also easily clarified. However, even in the case where the antibody having such reactivity is used, it was extremely difficult to exceed the detection sensitivity of a conventional product.

Therefore, the present inventors have conducted intensive studies in order to develop a test kit that is excellent in exceeding the detection sensitivity of a conventional product, and thus surprisingly have found that the detection sensitivity of a test kit for influenza A virus can be drastically improved when an antibody having excellent affinity for a native influenza A virus nuclear protein but not causing an antigen-antibody reaction by Western blotting with a full-length influenza A virus nuclear protein separated using SDS-PAGE is used as the antibody immobilized to a chromatography medium.

That is, the present invention relates to a kit for detecting influenza A virus to which a principle of immunochromatography is applied, and in which the antibody immobilized to a chromatography medium is an antibody causing an antigen-antibody reaction with a native influenza A virus nuclear protein but not causing an antigen-antibody reaction by Western blotting with a full-length influenza A virus nuclear protein separated using SDS-PAGE.

### Summary of the Invention

Hereinafter, the present invention will be described in more detail as follows.
(1) A kit for detecting influenza A virus by immunochromatography, containing: a chromatography medium in which a first antibody causing an antigen-antibody reaction with an influenza A virus nuclear protein but not causing an antigen-antibody reaction with an influenza B virus nuclear protein is immobilized; and a labeling reagent in which a second antibody causing an antigen-antibody reaction with an influenza A virus nuclear protein but not causing an antigen-antibody reaction with an influenza B virus nuclear protein is conjugated with a labeling substance,
   wherein the first antibody is an antibody not causing an antigen-antibody reaction by Western blotting with a full-length influenza A virus nuclear protein separated using SDS-polyacrylamide gel electrophoresis, and the second antibody is an antibody causing an antigen-antibody reaction by Western blotting with a full-length influenza A virus nuclear protein separated using SDS-polyacrylamide gel electrophoresis.
(2) The kit for detecting influenza A virus by immunochromatography according to (1), wherein the first antibody is one or more monoclonal antibodies.
(3) The kit for detecting influenza A virus by immunochromatography according to (1) or (2), wherein the first antibody is an antibody obtained by immunizing a full-length nuclear protein of influenza A virus subtype H1N1.
(4) A method for detecting influenza A virus by immunochromatography, using: a chromatography medium in which a first antibody causing an antigen-antibody reaction with an influenza A virus nuclear protein but not causing an antigen-antibody reaction with an influenza B virus nuclear protein is immobilized; and a labeling reagent in which a second antibody causing an antigen-antibody reaction with an influenza A virus nuclear protein but not causing an antigen-antibody reaction with an influenza B virus nuclear protein is conjugated with a labeling substance,
   wherein the first antibody is an antibody not causing an antigen-antibody reaction by Western blotting with a full-length influenza A virus nuclear protein separated using SDS-polyacrylamide gel electrophoresis, and the second antibody is an antibody causing an antigen-antibody reaction by Western blotting with a full-length influenza A virus nuclear protein separated using SDS-polyacrylamide gel electrophoresis.
(5) The method for detecting influenza A virus by immunochromatography according to (4), wherein the first antibody is one or more monoclonal antibodies.
(6) The method for detecting influenza A virus by immunochromatography according to (4) or (5), wherein the first antibody is an antibody obtained by immunizing a full-length nuclear protein of influenza A virus subtype H1N1.

### Advantageous Effects of Invention

In the test kit for influenza A virus of the present invention, the detection sensitivity is high, therefore, the determination of "positive" can be obtained using less amount of virus than that in a conventional test kit, thus the determination of false negative is decreased, and the reliability of the determination of "negative" becomes extremely high. Therefore, useful information can be provided for a doctor to make an accurate diagnosis of influenza virus infection for a patient in an initial stage of infection and soon after the start of virus proliferation in the living body, and thus the treatment with an anti-influenza virus agent can be started at an early stage. In addition, even for a patient in which the proliferation rate of virus is suppressed by the inoculation of vaccine, the presence or absence of infection of the influenza virus can be accurately diagnosed, and thus the attention of spread of the infection can be drawn, further the information that is epidemiologically important in order to determine the effect of influenza vaccine can be provided.

### Brief Description of Drawings

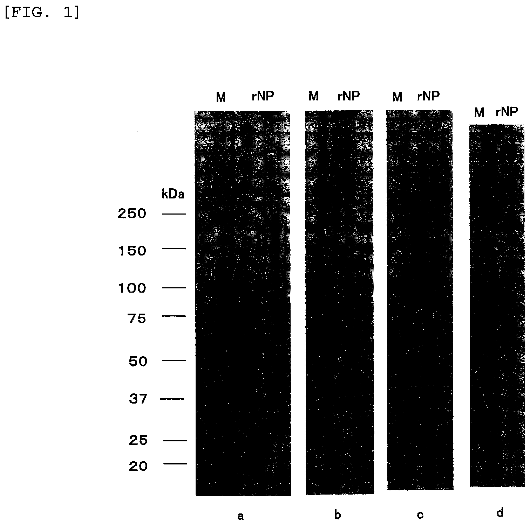

FIG. 1 shows reaction results of antibody 1C6, 6F7, or 10G5, and a recombinant nuclear protein of influenza A virus (56 kDa) by Western blotting. M in the upper part of FIG. 1 shows a lane in which a molecular weight marker has been run, and rNP shows a lane in which a recombinant nuclear protein has been run. (a) Shows a result of a reaction of a PVDF membrane to which a full-length recombinant nuclear protein separated using SDS-PAGE has been transferred with antibody 7307. A band is detected in the range of molecular weight of 50 to 75 kDa. The reactivity of antibody 1C6, 6F7 or 10G5 and a recombinant nuclear protein was examined under the conditions that the antigen-antibody reaction of antibody 7307 and a recombinant nuclear protein is confirmed. (b) Shows a result of antibody 1C6. (c) Shows a result of antibody 6F7. (d) Shows a result of antibody 10C5. The reaction of antibody 1C6, 6F7 or 10G5 and a recombinant nuclear protein was not detected under the conditions that the reactivity of antibody 7307 is confirmed.

### Description of Embodiments

The present invention is a kit for detecting influenza A virus by immunochromatography using the first antibody and second antibody that cause an antigen-antibody reaction with an influenza A virus nuclear protein but does not cause an antigen-antibody reaction with an influenza B virus nuclear protein, and which is characterized in that as the first antibody immobilized to a chromatography medium, an antibody causing an antigen-antibody reaction with a native influenza A virus nuclear protein but not causing an antigen-antibody reaction by Western blotting with a full-length influenza A virus nuclear protein separated using SDS-PAGE is used.

Each of the first antibody and the second antibody that are used in the present invention is an antibody causing an antigen-antibody reaction with an influenza A virus nuclear protein but not causing an antigen-antibody reaction with an influenza B virus nuclear protein. Influenza virus is classified into type A, type B, and the like according to the differences in the antigenicity of the nuclear protein. Further, influenza A virus has haemagglutinin (HA) and neuraminidase (NA), which are glycoproteins, on the surface of virus particles, and is classified into various subtypes according to the differences in the structure of these HA and NA. Each of the first antibody and the second antibody that are used in the present invention recognizes a nuclear protein of influenza virus, and thus is an antibody that can cause widely an antigen-antibody reaction with a nuclear protein of various subtypes of influenza A virus, and at least can cause an antigen-antibody reaction with a nuclear protein of subtype H1N1, subtype H3N2, subtype H5N1, and subtype H7N7 of influenza A virus, but does not cause an antigen-antibody reaction with a nuclear protein of influenza B virus. The influenza A virus nuclear protein with which the first antibody and second antibody of the present invention cause an antigen-antibody reaction may be a native protein separated from a virus, or may be a recombinant protein produced based on the nucleic acid sequence of the known nuclear protein gene. Further, the nuclear protein with which the first antibody and second antibody of the present invention cause an antigen-antibody reaction may be a nuclear protein separated and purified from a component of a virus or the unpurified nuclear protein, and if not separated, the nuclear protein may be a nuclear protein derived from a virus that is treated with a surfactant such that the nuclear protein is easily brought into contact with an antibody.

The "native influenza A virus nuclear protein" as used in the present invention may be a nuclear protein in which a conformational structure of a naturally existing influenza A virus nuclear protein, at least a conformational structure that is sufficient to maintain the antigen-antibody reaction with a specific antibody is left, and thus the nuclear protein in which a conformational structure of the naturally existing protein is destroyed by SDS-PAGE and the like, and the substantial antigen-antibody reaction of an influenzaAvirus nuclear protein with the antibody cannot be maintained is removed.

It can be confirmed by a well-known immunoassay method whether or not the first antibody and second antibody used in the present invention cause the antigen-antibody reaction with a nuclear protein of influenza virus. That is, when the immunoassay method is classified according to the measurement form, there are methods such as a sandwich method, a competition method, and an agglutination method, and when the immunoassay method is classified according to the label to be used, there are methods such as a fluorescence method, an enzyme method, and a radiation method. Any method among these immunoassay methods can be used for the confirmation of an antigen-antibody reaction. The description of "not causing an antigen-antibody reaction" as used in the present invention means that in the immunoassay method described above, the antigen-antibody reaction is not caused at a detectable level, or even if the antigen-antibody reaction is caused, the degree of the reaction is obviously weak as compared with the degree of the antigen-antibody reaction with an influenza A virus nuclear protein, and is the same degree as that with other proteins constituting influenza virus, thus is not the specific reaction.

The first antibody used in the present invention is an antibody causing an antigen-antibody reaction with an influenza A virus nuclear protein but not causing an antigen-antibody reaction with an influenza B virus nuclear protein, and further maybe an antibody not causing an antigen-antibody reaction with the protein in which the conformational structure at a site where the antigen-antibody reaction of a naturally existing influenza A virus nuclear protein is caused is destroyed. As such an antibody, for example, an antibody causing an antigen-antibody reaction with an influenza A virus nuclear protein but not causing an antigen-antibody reaction with an influenza B virus nuclear protein, and further not causing an antigen-antibody reaction by Western blotting with a full-length influenza A virus nuclear protein separated using SDS-PAGE is preferred.

The "SDS-polyacrylamide gel electrophoresis" as used in the present invention means a separation/analysis method of protein that is conventionally used in the technical field to which the present invention pertains, and can be performed in accordance with a method of representatively, Laemmli, U.K. (Nature, 227: 680-685 (1970)), but is not limited to this method. Specifically, the SDS-polyacrylamide gel electrophoresis can be performed, for example, by the following procedures. First, separation gel composed of polyacrylamide at a concentration of 10 to 15% is placed between plates, and on which concentrated gel composed of polyacrylamide at a concentration of 3 to 5% is overlaid, and the produced gel is attached to a slab type electrophoresis apparatus. Into a solution containing an influenza A virus nuclear protein, an equal parts of a 2-fold concentrated sample buffer (125 mM Tris-HCl, 20% glycerol, 2% SDS, 2% 2-mercaptoethanol, 0.001% bromophenol blue, and pH 6. 8) is added, and the resulting mixture is subjected to a heat treatment at 100°C for 5 to 10 minutes to obtain a sample for electrophoresis. The sample for electrophoresis and a commercially available molecular weight marker are added to a lane prepared in concentrated gel, respectively, and electrophoresis is performed at a constant current of 20 mA for 30 to 90 minutes by using a buffer for electrophoresis (192 mM glycine, 0.1% SDS, 24 mM Tris, and pH 8.3). A full-length influenza A virus nuclear protein separated using SDS-PAGE can be obtained as a band corresponding to a molecular weight of around 56 kDa in the separation gel.

The solution containing an influenza A virus nuclear protein to apply to SDS-PAGE is not limited to anything as long as the amount of the influenza A virus nuclear protein is sufficient to cause an antigen-antibody reaction with an antibody in a Western blotting that is performed finally, for example, 1 to 2 mg, and further the nuclear protein may be purified or unpurified. Examples of the solution containing an influenza A virus nuclear protein include, for example, a suspension of an influenza A virus, influenza HA vaccine available on the market, and a solution of a recombinant influenza A virus nuclear protein.

In consideration that the binding amount of SDS is around 1.2 to 1.5 per 1 of the protein, SDS in a 2-fold concentrated sample buffer used for SDS-PAGE can be used by appropriately changing the concentration in the range of 0.5 to 5% by weight depending on the amount of an influenza A virus nuclear protein. Further, 2-mercaptoethanol in a 2-fold concentrated sample buffer acts as a reducing agent that cleaves the disulfide bond present in an influenza A virus nuclear protein and may be used by appropriately changing the concentration in the range of 1 to 10% by weight, and instead of which a reducing agent composed of another substance such as dithiothreitol (DTT) can be used.

The "Western blotting" as used in the present invention can be performed by transferring a full-length influenza A virus nuclear protein separated using SDS-PAGE on a polyvinylidene difluoride (PVDF) membrane, for example, in accordance with a method of Towbin H., et al. (Proc. Natl. Acad. Sci. U.S.A., 76: 4350-4354 (1979)), but the Western blotting is not limited to this method. Specifically, a PVDF membrane is immersed in 100% methanol for 10 seconds, further in a transfer electrode buffer (192 mM glycine, 5% methanol, 25 mM Tris-HCl, and pH 8.3) for 30 minutes, and used for transfer. The transfer apparatus is assembled as follows: a filter paper, a PVDF membrane, gel in which SDS-PAGE has been completed, and a filter paper are overlaid in this order from the bottom on an anode electrode plate; and on which a cathode electrode plate is fixed. In addition, the filter paper is immersed in a transfer electrode buffer for 2 to 3 minutes in advance. The transfer is performed at a constant current of 1.9 mA/cm² for 60 to 90 minutes. The PVDF membrane after the transfer is completed is subjected to a blocking operation by incubation at room temperature for 60 minutes in a blocking solution (0.5% BSA, 10 mM Tris-HCl, 140 mMNaCl, 0.01% Tween20, and pH 7.5). After the blocking is completed, the PVDF membrane is incubated for 5 minutes twice in a washing buffer (10 mM Tris-HCl, 140 mM NaCl, 0.01% Tween20, and pH 7.5) and washed, then incubated at room temperature for 90 minutes with the anti-influenza A virus nuclear protein antibody as a primary antibody and reacted with the antibody. After the reaction with a primary antibody is completed, the PVDF membrane is incubated for 5 minutes twice in a washing buffer for wash, then incubated at room temperature for 60 minutes with a secondary antibody, for example, an antibody that is labeled with a labeling substance such as an enzyme, a fluorescent substance, or a radioactive isotope and is specifically reacted with a primary antibody. After the reaction with a secondary antibody is completed, the PVDF membrane is incubated for 5 minutes twice in a washing buffer for wash, then subjected to the detection in Western blotting by the visualization of the primary antibody that is bound to an influenza A virus nuclear protein transferred in the PVDF membrane in a way the nature of a labeling substance.

The first antibody used in the present invention is an antibody not causing an antigen-antibody reaction by Western blotting with a full-length influenza A virus nuclear protein separated using SDS-PAGE. Herein, the description of not causing an antigen-antibody reaction by Western blotting means that the antigen-antibody reaction is not caused at a detectable level under the conditions such as the antibody concentration, the antigen concentration, the substrate concentration, or the reaction time, in the standard Western blotting, or means that the antigen-antibody reaction is not caused specifically only with the influenza A virus nuclear protein while binding also to a protein other than the influenza A virus nuclear protein. The confirmation that the first antibody used in the present invention does not cause the antigen-antibody reaction by Western blotting with a full-length influenza A virus nuclear protein separated using SDS-PAGE can be performed such as the following. A commercially available antibody which is confirmed that causes the antigen-antibody reaction with an influenza A virus nuclear protein by Western blotting, for example, item stock number 7307 (manufactured by Medix Biochemica) is used as a positive control antibody. The first antibody is determined whether it cannot detect the influenza A virus nuclear protein under the conditions that the positive control antibody causes an antigen-antibody reaction with an influenza A virus nuclear protein on a PVDF membrane and can detect the nuclear protein. The first antibody used in the present invention may be an antibody not causing an antigen-antibody reaction with an influenza A virus nuclear protein in the Western blotting at the same antibody concentration that a positive control antibody can detect the influenza A virus nuclear protein. The first antibody is preferably an antibody not causing the reaction with an influenza A virus nuclear protein at twice the concentration of the positive control antibody, and is more preferably an antibody not causing an antigen-antibody reaction with an influenza A virus nuclear protein at 5 times or 10 times the concentration of the positive control antibody. Further, the first antibody used in the present invention may be an antibody not causing an antigen-antibody reaction with an influenza A virus nuclear protein in the Western blotting at the same antigen concentration that a positive control antibody can detect the influenza A virus nuclear protein. The preferred first antibody is an antibody not causing the reaction at twice the antigen concentration that a positive control antibody can detect, and the more preferred first antibody is an antibody not causing an antigen-antibody reaction with an influenza A virus nuclear protein at 5 times or 10 times the antigen concentration that a positive control antibody can detect.

The first antibody used in the present invention can be produced by the administration of an influenza A virus nuclear protein as an immunogen to an animal such as a mouse, a rat, a guinea pig, a canine, a goat, an ovine, a swine, a horse, and a bovine. The influenza A virus nuclear protein used as an immunogen is not particularly limited to in any form as long as the nuclear protein is present in a large amount and the function as an immunogen is exerted, however, for example, includes a suspension of influenza A virus, influenza HA vaccine available on the market, and a solution of a recombinant influenza A virus nuclear protein. In order to suppress the high immunogenicity of the HA and NA contained in an immunogen, an immunogen may be used after nuclear protein purification by ultracentrifugation (see, for example, J. Biochem., 102: 1241-1249 (1987)) or a protease treatment (see, for example, J. Immunol. Methods, 180: 107-116 (1995)). The first antibody used in the present invention is an antibody specifically causing an antigen-antibody reaction with an influenza A virus nuclear protein but not substantially causing an antigen-antibody reaction with a full-length influenza A virus nuclear protein separated using SDS-PAGE. Therefore, an influenza A virus nuclear protein used as the immunogen is preferably the protein that has not been treated with a sample buffer for SDS-PAGE containing a reducing agent, is more preferably the protein that has not been treated with SDS that is an anionic surfactant, and is furthermore preferably a native influenza A virus nuclear protein. A preferred immunogen for the first antibody used in the present invention is suspensions of influenza A virus in a buffer not containing substances such as an anionic surfactant, or a full-length recombinant influenza A virus nuclear protein.

In the case where the first antibody used in the present invention is a polyclonal antibody, for example, the immunogen can be prepared as the following. An antiserum is prepared from an animal immunized with the influenza A virus nuclear protein described above, and the antiserum is purified, for example, by affinity chromatography using a carrier to which an influenza A virus nuclear protein is bound, and then the antiserum or an immunoglobulin fraction causing an antigen-antibody reaction with an influenza A virus nuclear protein but not causing an antigen-antibody reaction with an influenza B virus nuclear protein is obtained. The antiserum or the immunoglobulin fraction is incubated with a full-length influenza A virus nuclear protein transferred on a PVDF membrane after SDS-PAGE separation, thus an antibody against the nuclear protein in the antiserum binds to the full-length influenza A virus nuclear protein on a PVDF membrane, and the antibody is separated and removed from the antiserum or the immunoglobulin fraction, accordingly an polyclonal antibody that can be used as the first antibody of the present invention can be prepared.

In the case where the first antibody used in the present invention is one or more monoclonal antibodies, for example, spleen cells are collected from an animal immunized with the influenza A virus nuclear protein described above, then the obtained spleen cells are subjected to the cell fusion with a tumor cell such as a myeloma cell in accordance with a known technique (see, for example, Nature, 256 : 495-497 (1975)), and thus a hybridoma producing the first antibody used in the present invention can be obtained.

As the method for screening for the hybridoma producing the first antibody, for example, the following procedures can be performed.

In the primary screening, the hybridomas are screened for an antibody causing an antigen-antibody reaction with an influenza A virus nuclear protein but not causing an antigen-antibody reaction with an influenza B virus nuclear protein in the culture supernatant.

The primary screening can be performed by a solid phase ELISA method using a purified influenza A virus nuclear protein or a recombinant influenza A virus nuclear protein as an antigen. The antigen is adsorbed to solid-phase carrier such as a microtiter plate,magnetic particles, a nitrocellulose membrane. The antigen adsorbed to the solid phase is brought into contact with the culture supernatant of the hybridoma, and the antibody that becomes to indirectly bind to the solid phase and then is detected by using an antibody labeled with a labeling substance, or the like. In the screening for the intended antibody, an influenza B virus nuclear protein can be used as a negative control antigen.

As another method of the primary screening, an antibody in a culture supernatant of hybridoma is directly or indirectly immobilized to solid-phase carrier, and then an influenza A virus nuclear protein is brought into contact with the antibody as an antigen. An antibody causing an antigen-antibody reaction with an influenza A virus nuclear protein can be detected by the direct labeling of the antigen or by the indirect labeling of the antigen using a specific antibody or the like.

The primary screening for the antibody used in the present invention can be performed by any method in which an antigen or an antibody is immobilized to a solid phase. Further, a rough selection is performed by using a solid phase to which an antigen is adsorbed, and then a more precise selection can be performed by using a solid phase to which an antibody is immobilized.

The first antibody used in the present invention is an antibody that is immobilized to a chromatography medium and used, therefore, the preferable method of the primary screening is a method in which an antibody in a culture supernatant of a hybridoma is directly or indirectly immobilized to a membranous solid carrier and then an influenza A virus nuclear protein is brought into contact with the antibody since the immobilized antibody in the screening method is similar to that in the embodiment of the invention.

Antibodies obtained from the primary screening, which causing an antigen-antibody reaction with the influenza A virus nuclear protein but not causing an antigen-antibody reaction with an influenza B virus nuclear protein, are subjected to the secondary screening. In the secondary screening, the selected hybridoma is a hybridoma producing an antibody not causing an antigen-antibody reaction in Western blotting with a full-length influenza A virus nuclear protein separated using SDS-PAGE. As described above, the full-length influenza A virus nuclear protein separated using SDS-PAGE is transferred onto a PVDF membrane. A culture supernatant of the hybridoma selected in the primary screening is brought into contact with the PVDF membrane, and the detection is performed in Western blotting as described above, as a result, a hybridoma producing the intended antibody can be selected.

As described in detail in the following Examples, for 6 hybridomas that had been selected in the primary screening and caused a particularly strong antigen-antibody reaction with an influenza A virus nuclear protein, the secondary screening was performed by selecting an antibody not causing an antigen-antibody reaction by Western blotting with a full-length influenza A virus nuclear protein separated using SDS-PAGE and thus 3 hybridomas are selected. The monoclonal antibodies produced by these hybridomas were immobilized to a chromatography medium, and the detection of an influenza A virus was performed, as a result, in all of the 3 monoclonal antibodies, the determination of positive could be obtained with the detection sensitivity exceeding that of a conventional product. That is, multiple hybridomas producing a monoclonal antibody that is preferable as the first antibody used in the present invention could be selected.

The first antibody used in the present invention can be prepared as follows: each hybridoma described above is cultured in a culture medium that is usually used for a cell culture, and the first antibody of the present invention is recovered from the culture supernatant. In addition, the first antibody used in the present invention can also be prepared as follows: each hybridoma described above is administered into an abdominal cavity of the animal from which the hybridoma is derived, the ascites is retained, and the first antibody used in the present invention is recovered from the ascites.

The second antibody used in the present invention is an antibody causing an antigen-antibody reaction with an influenza A virus nuclear protein but not substantially causing an antigen-antibody reaction with an influenza B virus nuclear protein, and may be an antibody having high affinity to an influenza A virus nuclear protein. In the preferred embodiment, according to the combination with the first antibody, an antibody causing an antigen-antibody reaction by Western blotting with a full-length influenza A virus nuclear protein separated using SDS-PAGE is used.

The second antibody used in the present invention may be a polyclonal antibody or a monoclonal antibody. In the case of a monoclonal antibody, the second antibody may be a single kind of antibody, or may be a mixture of multiple kinds of antibodies. Further, in the case where a monoclonal antibody is used as the antibody in the present invention, the antibody can also be used as a fragment having affinity with an antigen such as Fab, or F(ab')2.

The second antibody used in the present invention can be prepared by using an immunogen that is for the production of the first antibody described above. In the case where the second antibody of the present invention is a polyclonal antibody, blood is collected from the immunized animal, an antiserum causing an antigen-antibody reaction with an influenza A virus nuclear protein but not causing an antigen-antibody reaction with an influenza B virus nuclear protein or an immunoglobulin fraction in an antiserum is purified as described above, and thus the second antibody of the present invention can be produced. In the case where the second antibody used in the present invention is a monoclonal antibody, a hybridoma is produced by a known method, a hybridoma producing an antibody causing an antigen-antibody reaction with an influenza A virus nuclear protein but not causing an antigen-antibody reaction with an influenza B virus nuclear protein in a culture supernatant is screened as described above, and thus the second antibody used in the present invention can be produced. In the case where an antibody causing an antigen-antibody reaction by Western blotting with a full-length influenza A virus nuclear protein separated using SDS-PAGE is used as the second antibody in the present invention, the secondary screening by Western blotting is performed according to the procedures described above, an antibody in which an antigen-antibody reaction is detected is selected, and thus the intended antibody can be selected.

The detection kit of the present invention contains a chromatography medium to which the first antibody described above is immobilized. In the present invention, the first antibody immobilized to a chromatography medium forms a determination site. The chromatography medium used in the present invention is an inactive one composed of a fine porous substance showing capillarity, the material of which is not particularly limited as long as the material does not react with a labeling reagent, a component in the biological sample, and the like, and a known one can be used. Specifically, examples of the chromatography medium include a cellulose derivative such as nitrocellulose, and cellulose acetate; a nylon membrane; filter paper; and glass fiber filter paper.

The form and size of the chromatography medium is not particularly limited, and any chromatography medium may be used as long as the medium is appropriate in terms of the actual operation and the observation of the results. In order to perform the operation more simply, a support composed of a material such as plastic can be provided on the back surface of a chromatography medium. The properties of this support are not particularly limited, but in the case where the observation of the detection results is performed with visual determination, the support has preferably a color that is not similar to the color provided by the labeling substance, and has more preferably colorless or white usually.

In the chromatography medium, features such as a sample adding site to which a biological sample is added (such as a sample pad); a site from which a solid component in a sample is removed (such as a solid component separating site); a developer adding site to which a developer is added; an absorbing site in which a labeling reagent and a developer that have not captured in a determination site are sucked up (such as an absorption pad); a control site showing that the detection is normally performed; may be arbitrarily incorporated. The members of these sites are not particularly limited as long as a sample solution or a developer can be moved by capillarity, and generally the members are selected from multiple porous substances such as a nitrocellulose membrane, filter paper, and glass fiber filter paper, depending on the intended purpose and used to be arranged so as to be connected by capillary with a chromatographymediumto which the first antibody is immobilized.

As the method of immobilizing the first antibody to a chromatography medium, there are a method of directly immobilizing the first antibody to a chromatography medium by a physical or chemical means, and a method of indirectly immobilizing the first antibody to a chromatography medium by binding the first antibody physically and chemically to a fine particle such as a latex particle and capturing the fine particle in a chromatography medium to immobilize the first antibody there; however, from the view point of the ease of sensitivity adjustment, the method of directly immobilizing the first antibody to a chromatography medium is preferable. As the method of directly immobilizing the first antibody to a chromatography medium, physical adsorption may be used, or covalent binding maybe used. Generally, in the case where a chromatography medium is a nitrocellulose membrane or a mixed nitrocellulose ester membrane, the physical adsorption can be performed. In the case where covalent binding is used, the activation of the chromatography medium is performed by the use of a comound such as cyanogen bromide, glutaraldehyde, and carbodiimide. The chromatography medium and the first antibody can be adsorbed or bound to each other by a method of, for example, a microsyringe, a pen with an adjustment pump, and ink jet print. The form of the determination site is not particularly limited, but the determination site can be formed in a form of a circular spot, a line extending perpendicular to the development direction of a chromatography medium, a number, a letter, or a symbol such as +, and -.

As needed, a chromatography medium to which the first antibody is immobilized is subjected to a blocking treatment. Examples of the blocking agent that can be used for the blocking treatment include a protein such as bovine serum albumin, skim milk, casein, and gelatin, and further a blocking agent available on the market such as Blocking Peptide Fragment (manufactured by TOYOBO CO., LTD.), and a hydrophilic high molecular polymer.

The detection kit of the present invention contains a labeling substance to which the second antibody described above and a labeling substance are conjugated. As the labeling substance used in the present invention, an enzyme or an insoluble carrier can be used. As the enzyme, there are alkaline phosphatase, horseradish peroxidase, β-galactosidase, urease, glucose oxidase, and the like, and these can be used together with a known chromogenic substrate corresponding to each enzyme. As the insoluble carrier, materials such as a metal particle in a colloidal state, such as gold, silver, and platinum; a metal oxide particle in a colloidal state, such as iron oxide; a nonmetal particle in a colloidal state, such as sulfur; a latex particle composed of a synthetic polymer; can be used. Examples of the metal particle in a colloidal state and the metal oxide particle in a colloidal state include, for example, a gold particle in a colloidal state, a silver particle in a colloidal state, a platinum particle in a colloidal state, an iron oxide particle in a colloidal state, and an aluminum hydroxide particle in a colloidal state. In particular, a gold particle in a colloidal state and a silver particle in a colloidal state are preferable in the point that the gold particle in a colloidal state shows red, and the silver particle in a colloidal state shows yellow, when the particle diameter is appropriate. The average particle diameter of these metal particles in a colloidal state is 1 nm to 500 nm, preferably 10 nm to 150 nm with which particularly strong color tone is obtained, and more preferably in the range of 40 nm to 100 nm. The labeling substance used for a labeling reagent of the present invention is preferably an insoluble carrier, more preferably a metal particle in a colloidal state, and furthermore preferably a gold particle in a colloidal state.

When, for example, a gold particle in a colloidal state is used as the metal particle in a colloidal state, the gold particle in a colloidal state available on the market may be used. Alternatively, a conventional method, for example, a method in which chloroauric acid is reduced with sodium citrate, can be used to prepare the gold particle in a colloidal state.

As the method in which the second antibody used in the present invention is conjugated with a labeling substance, a known method of physical adsorption or chemical bond can be used. For example, when the second antibody is labeled with a gold particle in a colloidal state, the second antibody is added into a solution in which gold particles are dispersed in a colloidal state and physically adsorbed with the gold particle, then a substance such as bovine serum albumin solution, and the above-describedblocking agent available on the market, are added to block the particle surface to which an antibody has not been conjugated, and thus the labeling is prepared.

The labeling reagent of the present invention can be included in a kit of the present invention as another reagent separate from the chromatography medium, however, a labeling reagent retaining portion is provided on the chromatography medium, and in which the labeling reagent can be dried and retained. When the labeling reagent is retained in the labeling reagent retaining portion, the labeling reagent is preferably retained such that the labeling reagent is promptly dissolved in a developer and moved freely by a capillary action. In order to improve the resolubility of labeling reagent, a saccharide such as saccharose, sucrose, trehalose, maltose, and lactose, and a sugar alcohol such as mannitol are added into the labeling reagent and coated, or these substances can be coated in advance, into the labeling reagent retaining portion. The labeling reagent retaining portion can be formed by the direct coating of a labeling reagent to a chromatography medium and then the drying, or a labeling reagent is coated to another porous substance separate from the chromatography medium, for example, cellulose filter paper, glass fiber filter paper, and nylon non-woven fabric, and dried to form a labeling reagent retaining member, then the chromatography medium and the retaining member can be arranged so as to be connected with capillary.

The biological sample that can be applied to a detection kit of the present invention is not particularly limited as long as it is suspected to contain the influenza A virus, but examples of the preferred sample include a nasal swab, a nasal aspirate, and a throat swab. These biological samples can be applied as it is to a kit of the present invention, however, the sample is usually suspended in or diluted with a developer to be applied.

The developer used together with a detection kit of the present invention, ingeneral, contains preferably a buffer agent such as phosphate, tris hydroxymethyl aminomethane hydrochloride, HEPES, and a Good's buffer, and an inorganic salt such as sodium chloride, using water as a solvent. Further, as needed, compounds such as a protein component such as bovine serum albumin (BSA), and an antiseptic agent may be contained. Furthermore, the developer used in the present invention may contain a nonionic surfactant such that the virus particle of influenza virus is destroyed, and the first antibody and second antibody of the present invention are easily brought into contact with the nuclear protein. Examples of the nonionic surfactant to be added into a developer include, for example, polyoxyethylene alkyl ether, polyoxyethylene sorbitan fatty acid ester (trade name "Tween" series), polyoxyethylene p-t-octylphenyl ether (trade name "Triton" series), and polyoxyethylene p-t-nonylphenyl ether (trade name "Triton N" series), but the nonionic surfactant is not limited to these. The content of these nonionic surfactants is not particularly limited, but these nonionic surfactants are used in the range of 0.01 to 10.0% by weight, preferably 0.1 to 5.0% by weight, more preferably 0.1 to 1.0% by weight, and furthermore preferably 0.3 to 1.0% by weight, relative to the weight of the entire developer.

Using a detection kit of the present invention, influenza A virus can be detected, for example, by the following operation.

In one embodiment of the present invention, a biological sample collected from a subject is mixed with a labeling reagent in a developer in advance, a complex of a nuclear protein and a labeling reagent is formed, and then the complex is brought into contact with a chromatography medium. The developer containing a nuclear protein-labeling reagent complex moves in a chromatography medium as a mobile phase. When the nuclear protein-labeling reagent complex moves in a determination site of the chromatography medium, the immobilized first antibody captures the complex, and the labeling reagent is indirectly bound to the determination site. The detection or determination of influenza A virus can be performed by the observation of the color development intensity of the labeling reagent present in a determination site visually or using a densitometer and the like, as follows: when the labeling substance is an insoluble carrier, the intensity is detected directly for the carrier; or when the labeling substance is an enzyme, the intensity is detected for the reaction product after the enzyme reacts with a substrate.

In a further embodiment of the present invention, the detection of influenza A virus can be performed using a chromatography medium having a labeling reagent retaining portion. When the biological sample and the developer are brought into contact with a chromatography medium, these biological sample and developer move in the chromatography medium as a mobile phase, and dissolve the labeling reagent retained in the labeling reagent retaining portion. The labeling reagent dissolved in a mobile phase forms a complex with an influenza A virus nuclear protein in a sample, and moves in the chromatography medium. The nuclear protein-labeling reagent complex that has reached the determination site of the chromatography medium is captured in the first antibody immobilized to the determination site, and thus the labeling reagent is indirectly bound to the determination site. The detection or determination of influenza A virus can be performed by the measurement of the labeling reagent present in the determination site visually, or using, for example, a densitometer.

Hereinafter, the present invention will be explained more specifically with reference to Examples, however, should not be construed to be limited to the Examples.

### Examples

### (Example 1) production of anti-influenza A virus nuclear protein antibody

A recombinant nuclear protein produced based on the amino acid sequence of a nuclear protein derived from influenza A virus A/Puerto Rico/8/34 (H1N1) strain (DDBJ/GenBank database, Accession No. V01084) was used as the immunogen. An equal parts of complete Freund' s adjuvant was added into and completely mixed with the immunogen, and then a BALB/c mouse was immunized 4 times in total at 2-week intervals. Spleen cells were collected from the immunized mouse three days after the last immunization, and hybridomas were produced by the fusion of the spleen cells with myeloma cells (P3U1) using a known standard technique. 10 to 15 days after the production of the hybridomas, the screening for an antibody that is specific to an influenza A virus nuclear protein was performed using a culture supernatant of the hybridoma.

In the primary screening, an antibody causing an antigen-antibody reaction with the influenza A virus nuclear protein but not causing an antigen-antibody reaction with an influenza B virus nuclear protein was selected by the performing of the following screening operations in series.

First, according to a solid phase ELISA method, the screening was performed using a microtiter plate as the solid phase, and using recombinant nuclear protein, which is the same protein as an immunogen, as an antigen. That is, 100 µL of a solution containing 10.0 µg/mL of a recombinant nuclear protein in a carbonic acid buffer solution was added in each well of a 96-well plate (SUMILON), and incubated at 4°C overnight, and thus the antigen was immobilized. Next, each well was washed with PBS containing 0.1% Tween 20 (trade name) (hereinafter, referred to as PBS-Tween), 1% BSA diluted with PBS was added into the well, and the well was blocked at 4°C overnight. Each well was washed with PBS-Tween, then 100 µL of a culture supernatant was added into the well, and the well was incubated at 37°C for 1 hour. Each well was washed with PBS-Tween thoroughly, then an alkaline phosphatase labeled anti-mouse Igs antibody (manufactured by Funakoshi Co., Ltd.) diluted 1000 fold was added into each well, and the well was incubated at 37°C for 1 hour. Each well was washed with PBS-Tween thoroughly, then 100 µL of p-nitrophenyl phosphate was added into each well as a substrate, and the well was incubated at room temperature for 30 minutes. 100 µL of a reaction stop solution was added into each well, and the color developing level was measured at a wavelength of 405 nm. Using a culture supernatant corresponding to a well showing high color development, the screening operation was further performed.

Next, using a nitrocellulose membrane as the solid phase, and using an influenza A virus as the antigen, the screening was performed with a measurement system of immunochromatography. That is, a culture supernatant was applied onto a nitrocellulose membrane having a size of 35 mm × 5 mm, and dried at 37°C for 1 hour, as a result, the antibody was immobilized to form a determination line. In addition, a culture supernatant and a gold colloidal solution were mixed in a 50 mM HEPES buffer solution, and thus an antibody-sensitized gold colloidal solution was produced. An influenza A virus A/New Caledonia/20/99 (H1N1) strain, or an influenza B virus B/Tokio/53/99 strain as a negative control was suspended in a sample diluent (20 mMphosphate buffer solution (pH 7.4), 0.3% skim milk, 0.3% Tween 20, and 0.15 M sodium chloride), and the suspension was added into each well of a 96-well plate (SUMILON). Further, the antibody-sensitized gold colloidal solution described above was added into each well, and was mixed well with the virus suspension. Into the mixture in each well, the end of the nitrocellulose membrane was inserted, and thus a mixture containing viruses was developed. The nitrocellulose membrane was taken out from the mixture 10 minutes after the development, the color development intensity of the gold colloid captured in the determination line was measured with an immunochromato reader (manufactured by Hamamatsu Photonics K.K.). When the color development intensity exceeds 8.0mABs, the result was determined to be positive. The antibody used in the measurement system in which a positive result was obtained when the influenza A virus was developed and a negative result was obtained when the influenza B virus was developed, was selected as an antibody causing an antigen-antibody reaction with an influenza A virus nuclear protein but not causing an antigen-antibody reaction with an influenza B virus nuclear protein.

For the antibody selected in the primary screening, the secondary screening was performed by Western blotting using the recombinant nuclear protein described above, and an antibody causing an antigen-antibody reaction with an influenza A virus nuclear protein but not causing an antigen-antibody reaction with an influenza A virus nuclear protein separated using SDS-PAGE was selected. That is, 0.01 mg/mL of the recombinant nuclear protein described above was mixed with an equal parts of a 2× Tris-Glycine SDS Sample Buffer (manufactured by TEFCO) with 10% 2-mercaptoethanol, the resultant mixture was heated at 100°C for 10 minutes, and subjected to SDS-PAGE. The SDS-PAGE was performed in accordance with a known standard method by using Ready Gel J5-20% 12 well (manufactured by BIO-RAD). After the electrophoresis a protein was transferred from the gel to a Sequi-Blot PVDF Membrane (manufactured by BIO-RAD) with a blotting apparatus (manufactured by BIO-RAD). The PVDF membrane after the transfer was blocked with immunoblock (DS Pharma Laboratories) at room temperature for 1 hour. The blocking solution was removed, and the PVDF membrane was washed with PBS containing 0.05% Tween 20 (trade name) (hereinafter, referred to as T-PBS) for 10 minutes three times, then the resultant PVDF membrane was incubated at room temperature for 1 hour together with a culture supernatant containing the antibody selected in the primary screening. After washing with T-PBS for 10 minutes three times, the PVDF membrane was incubated at room temperature for 30 minutes with alkaline phosphatase labeled anti-mouse IgG (manufactured by SIGMA) that is diluted 5000 fold with T-PBS. After washing with T-PBS for 10 minutes three times, the PVDF membrane was incubated with 1-Step™ NBT/BCIP (manufacturedby PIERCE) that is a chromogenic substrate, and the antibody bound to the PVDF membrane was visualized. A commercially available anti-influenza A virus monoclonal antibody (item stock number 7307, manufactured by Medix Biochemica) is used as the positive control. When the binding of the antibody contained in a culture supernatant cannot be detected under the condition that the binding of the positive control antibody to a PVDF membrane can be detected visually, the antibody was selected as the antibody not causing an antigen-antibody reaction with an influenza A virus nuclear protein separated using SDS-PAG. The hybridoma producing the antibody was cloned, then three independent clones were selected, and named hybridoma 1C6, hybridoma 6F7, and hybridoma 10G5, respectively. In addition, the antibodies produced by hybridoma 1C6, hybridoma 6F7, and hybridoma 10G5 were named antibody 1C6, antibody 6F7, and antibody 10G5, respectively. The subclass of the monoclonal antibody obtained from each of the three hybridoma strains was all IgG1.

Reaction results of antibodies 1C6, 6F7, and 10G5 in Western blotting with a recombinant nuclear protein of influenza A virus are shown in FIG. 1. Each concentration of the antibodies was adjusted to 10 µg/mL, and each of the antibodies was reacted with 1.0 µg of a recombinant nuclear protein per lane. As shown in FIG. 1, a band of 56 kDa corresponding to a full-length influenza A virus nuclear protein was detected by antibody 7307, however, when antibody 1C6, 6F7, or 10G5 was usedunder the same conditions, the band could not be detected. Further, the amount of the recombinant nuclear protein was increased up to 5.0 µg per lane and the same experiment was performed again, however, in the case where the antibody 1C6, 6F7, or 10G5 was used, the detectable band was not confirmed by Western blotting (data not shown).

### (Example 2) Production of test kit by immunochromatography

### (1) Production of diluent for capture antibody

Isopropyl alcohol was mixed with 50 mM phosphate buffer solution (pH 7.4) so as to be diluted to 5%, and thus a diluent for the first antibody was prepared.

### (2) Production of determination site on chromatography medium

One antibody, or two antibodies in combination were selected among the antibodies 1C6, 6F7, and 10G5, and diluted with a diluent for a capture antibody so as to have the total antibody concentration of 1.0 mg/mL. The antibody solution was applied on a nitrocellulose membrane (manufactured by Millipore) having a size of 25 × 2.5 cm using an applicator (manufactured by BioDot), and dried at 50°C for 5 minutes, then further dried at room temperature for 1 hour, as a result, a determination site was prepared on a chromatography medium.

### (3) Production of labeling antibody solution

A gold colloidal suspension (manufactured by TANAKA KIKINZOKU KOGYO K.K.: average particle size of 40 nm, and gold concentration of 0.36 mM) was used as the labeling substance. Antibody 7307 alone, or a combination of antibody 7307 and any one of antibodies 1F6, 6G7, and 10G5 was diluted with a phosphate buffer solution (pH 7.4) so as to have the total antibody concentration of 0.05 mg/mL. 0.1 mL of antibody solution was added into 0.5 mL of gold colloidal suspension, and the mixture was left to stand at room temperature for 10 minutes. Next, into the resultant mixture, 0.1 mL of a phosphate buffer solution (pH 7.4) containing 1% BSA was added, and further the mixture was left to stand at room temperature for 10 minutes. After that, the mixture was stirred thoroughly, and subjected to centrifugation at 8000 × g for 15 minutes. The supernatant was removed, and 2 mL of a phosphate buffer solution (pH 7.4) containing 0.5% BSA was added into the resultant mixture.

### (4) Production of test kit by immunochromatography

The labeling antibody solution produced in the above (3) was uniformly added into a glass fiber pad (manufactured by Millipore) having a size of 15 mm × 300 mm, then dried with a vacuum dryer, as a result, a conjugation pad was produced. Next, a chromatography medium produced in the above (2) was bonded to a base material composed of a backing sheet, and further a conjugation pad, and a sample pad (manufactured by Millipore: 300 mm × 30 mm) that is a sample adding site were bonded successively in the upstream of the developing direction, an absorbent pad was bonded in the downstream of the developing direction, then the bonded chromatography medium was cut into a piece having a width of 5 mm, as a result, a test kit by immunochromatography was produced. The size of the absorbent pad per kit was 26 mm × 5 mm, and the gold content in the labeling antibody solution used was 1 µg.

### (5) Preparation of developer

Reagents were added into ultrapure water such that each concentration of the reagents was as follows: 10% Tween 20 was 1%, 0.1 M magnesium sulfate was 5 mM, dimethyl sulfoxide was 0.95%, 20% dextran sulphate sodium (weight-average molecular weight: 500,000) was 2%, and CE510 (manufactured by JSR Corporation) was 2%, and mixed. Further, sodium azide was added and mixed as an antiseptic agent so as to be 0.05%, and thus a developer was produced.

### (Example 3) Measurement of influenza A virus

Using a test kit produced in the above, a reactivity test with influenza A virus was performed according to the following method, and thus the performance of the test kit of the present invention was examined.

Nasal mucus was collected from a subject who had been determined to be negative in the infection test of influenza A virus (H3N2) using a PCR method. The collection of nasal mucus was performed as follows: one tube of a suction trap was inserted to the inner part of the nasal cavity of a subject, and the other tube was connected to a suction pump, and the suction pump was set to negative pressure to suck up the nasal mucus. The nasal mucus was diluted 20 fold with a developer, and thus an influenza A virus negative sample was prepared. An inactivated influenza A virus A/Panama/2007/99 (H3N2) was added to the negative sample, and thus an influenza A virus positive sample was prepared.

150 µL of each of a positive sample and a negative sample was placed on a sample pad of the test kit and developed, and 10 minutes later these samples were determined visually. When a red line was observed in the determination site, the result was expressed as "+"; when a red line was observed more strongly, the result was expressed as "++"; when a red line was observed but the color was extremely light, the result was expressed as "±"; and when a red line was not observed, the result was expressed as "-". The results are shown in Table 1.

### (Comparative Example 1)

In the production of a test kit in Example 2, except that the antibody to be applied in the determination site on a chromatography medium and the antibody to be bound to gold colloid were changed, the measurement of each of a positive sample and a negative sample was performed by the same operation as in Example 3.

As the antibody applying to the determination site of the chromatography medium, antibody 7307, antibody 1C6, or a combination thereof was used in place of any one of the antibodies 1C6, 6F7, and 10G5 or a combination thereof.

As the labeling antibody to be conjugated with gold colloid, antibody 6F7 or antibody 10G5 was used in place of the antibody 7307.

The results are shown in Table 1.

### (Comparative Example 2)

Using a commercially available test kit for influenza A virus by immunochromatography, ImunoAce Flu (trade name) (manufactured by TAUNS Laboratories, Inc.), the measurement of each of a positive sample and a negative sample was performed by the same operation as in Example 3.

The results are shown in Table 1.

**[Table 1]**

| | Immobilized antibody | Labeling antibody | Antigen concentration (*µ*g/mL) | | | | |
|---|---|---|---|---|---|---|---|
| | | | 0 | 10 | 20 | 40 | 80 |
| Example 3 | Antibody 1 C6 | Antibody 7307 | - | - | ± | + | ++ |
| | Antibody 1 C6 | Antibody 7307 | - | ± | + | ++ | ++ |
| | Antibody 10G5 | | | | | | |
| | Antibody 6F7 | Antibody 7307 | - | ± | + | ++ | ++ |
| | Antibody 10G5 | | | | | | |
| | Antibody 6F7 | Antibody 7307 | - | ± | + | ++ | ++ |
| | Antibody 10G5 | Antibody 1 C6 | | | | | |
| Comparative Example 1 | Antibody 7307 | Antibody 6F7 | - | - | - | ± | + |
| | | Antibody 10G5 | | | | | |
| | Antibody 7307 | Antibody 6F7 | - | - | - | ± | + |
| | Antibody 1 C6 | Antibody 10G5 | | | | | |
| | Antibody 1 C6 | Antibody 6F7 | - | - | - | - | + |
| | | Antibody 10G5 | | | | | |
| Comparative Example 2 | Unknown | Unknown | - | - | - | ± | + |

When an antibody causing an antigen-antibody reaction with an influenza A virus nuclear protein but not causing an antigen-antibody reaction with a nuclear protein separated using SDS-PAGE (antibody 1C6, 6F7, or 10G5) was used as the immobilized antibody of a test kit by immunochromatography, the test kit exhibited detection sensitivity for the influenza A virus several times higher than that of a test kit (Comparative Example 2) which is conventionally available on the market.

Particularly, in the case that the antibody causing an antigen-antibody reaction with a nuclear protein separated using SDS-PAGE (antibody 7307) was selected as a labeling antibody, and was used in a test kit in combination with the immobilized antibody described above, the test kit exhibited detection sensitivity higher than that of a conventional test kit.

On the other hand, when an antibody causing an antigen-antibody reaction also with a nuclear protein separated using SDS-PAGE (antibody 7307) was used as the immobilized antibody (Comparative Example 1), the detection sensitivity was equivalent to that of a conventional commercial product.

### (Example 4)

Except that inactivated influenza A virus A/New Caledonia/20/99 (H1N1) strain, A/Brisbane/10/2007 (H3N2) strain, orA/Solomon/03/2006 (H1N1) strain was used as the sample in place of the influenzaAvirus A/Panama/2007/99 (H3N2) strain, the measurement of the sample was performed by the same operation as in Example 3. As the immobilized antibody, antibody 6F7 or antibody 10G5 was used, and as the labeling antibody, antibody 7307 was used.

The results are shown in Table 2.

**[Table 2]**

| | Influenza virus strain | Antigen concentration (*µ*g/mL) | | | | |
|---|---|---|---|---|---|---|
| | | 0 | 10 | 20 | 40 | 80 |
| Example 4 | A/Panama/2007/99 (H3N2) | - | ± | + | ++ | ++ |
| | A/NewCaledonia/20/99(H1N1) | - | ± | + | ++ | ++ |
| | A/Brisbane/10/2007(H3N2) | - | ± | + | ++ | ++ |
| | A/Solomon/03/2006(H1N1) | - | ± | + | ++ | ++ |

The test kit of the present invention could detect an influenza A virus with high sensitivity in spite of the differences in the subtype.

### Industrial Applicability

The test kit for influenza A virus of the present invention has higher detection sensitivity of influenza A virus than that of a conventional test kit, therefore, the determination of "positive" can be obtained using less amount of virus. Accordingly, the test kit of the present invention has extremely high reliability for the determination of "negative" and it has an industrial applicability that the useful test kit can be provided.

## Claims

1. A kit for detecting influenza A virus by immunochromatography, comprising:
a chromatography medium in which a first antibody causing an antigen-antibody reaction with an influenza A virus nuclear protein but not causing an antigen-antibody reaction with an influenza B virus nuclear protein is immobilized; and
a labeling reagent in which a second antibody causing an antigen-antibody reaction with an influenza A virus nuclear protein but not causing an antigen-antibody reaction with an influenza B virus nuclear protein is conjugated with a labeling substance,
wherein the first antibody is an antibody not causing an antigen-antibody reaction with a full-length influenza A virus nuclear protein separated using SDS-polyacrylamide gel electrophoresis by Western blotting, and the second antibody is an antibody causing an antigen-antibody reaction by Western blotting with a full-length influenza A virus nuclear protein separated using SDS-polyacrylamide gel electrophoresis.

2. The kit for detecting influenza A virus by immunochromatography according to Claim 1,
wherein the first antibody is one or more monoclonal antibodies.

3. The kit for detecting influenza A virus by immunochromatography according to Claim 1 or 2,
wherein the first antibody is an antibody obtained by immunizing a full-length nuclear protein of influenza A virus subtype H1N1.

4. A method for detecting influenza A virus by immunochromatography, using:
a chromatography medium in which a first antibody causing an antigen-antibody reaction with an influenza A virus nuclear protein but not causing an antigen-antibody reaction with an influenza B virus nuclear protein is immobilized; and
a labeling reagent in which a second antibody causing an antigen-antibody reaction with an influenza A virus nuclear protein but not causing an antigen-antibody reaction with an influenza B virus nuclear protein is conjugated with a labeling substance,
wherein the first antibody is an antibody not causing an antigen-antibody reaction by Western blotting with a full-length influenza A virus nuclear protein separated using SDS-polyacrylamide gel electrophoresis, and the second antibody is an antibody causing an antigen-antibody reaction by Western blotting with a full-length influenza A virus nuclear protein separated using SDS-polyacrylamide gel electrophoresis.

5. The method for detecting influenza A virus by immunochromatography according to Claim 4,
wherein the first antibody is one or more monoclonal antibodies.

6. The method for detecting influenza A virus by immunochromatography according to Claim 4 or 5,
wherein the first antibody is an antibody obtained by immunizing a full-length nuclear protein of influenza A virus subtype H1N1.

## Patentansprüche

1. Kit zum Nachweisen von Influenza-A-Virus durch Immunchromatographie, umfassend:
ein Chromatographiemedium, in dem ein erster Antikörper, der eine Antigen-Antikörper-Reaktion mit einem Influenza-A-Virus-Kernprotein auslöst, jedoch keine Antigen-Antikörper-Reaktion mit einem Influenza-B-Virus-Kernprotein auslöst, immobilisiert wird; und
ein Markierungsreagens, in dem ein zweiter Antikörper, der eine Antigen-Antikörper-Reaktion mit einem Influenza-A-Virus-Kernprotein auslöst, jedoch keine Antigen-Antikörper-Reaktion auslöst mit einem Influenza-B-Virus-Kernprotein, konjugiert wird mit einer Markierungssubstanz,
wobei der erste Antikörper ein Antikörper ist, der keine Antigen-Antikörper-Reaktion mit einem vollständigen Influenza-A-Virus-Kernprotein auslöst, das unter Verwendung von SDS-Polyacrylamid-Gelelektrophorese durch Western-Blotting abgetrennt wird, und der zweite Antikörper ein Antikörper ist, der eine Antigen-Antikörper-Reaktion durch Western-Blotting mit einem vollständigen Influenza-A-Virus-Kernprotein auslöst, das abgetrennt wird unter Verwendung von SDS-Polyacrylamid-Gelelektrophorese.

2. Kit zum Nachweisen von Influenza-A-Virus durch Immunchromatographie nach Anspruch 1, wobei der erste Antikörper ein oder mehr monoklonale Antikörper ist.

3. Kit zum Nachweisen von Influenza-A-Virus durch Immunchromatographie nach Anspruch 1 oder 2, wobei der erste Antikörper ein Antikörper ist, der durch Immunisieren eines vollständigen Kernproteins von Influenza-A-Virus des Subtyps H1N1 erhalten wird.

4. Verfahren zum Nachweisen von Influenza-A-Virus durch Immunchromatographie, unter Verwendung von:
einem Chromatographiemedium, in dem ein erster Antikörper, der eine Antigen-Antikörper-Reaktion mit einem Influenza-A-Virus-Kernprotein auslöst, jedoch keine Antigen-Antikörper-Reaktion mit einem Influenza-B-Virus-Kernprotein auslöst, immobilisiert wird; und
einem Markierungsreagens, in dem ein zweiter Antikörper, der eine Antigen-Antikörper-Reaktion mit einem Influenza-A-Virus-Kernprotein auslöst, jedoch keine Antigen-Antikörper-Reaktion mit einem Influenza-B-Virus-Kernprotein auslöst, konjugiert wird mit einer Markierungssubstanz,
wobei der erste Antikörper ein Antikörper ist, der keine Antigen-Antikörper-Reaktion durch Western-Blotting mit einem vollständigen Influenza-A-Virus-Kernprotein auslöst, das abgetrennt wird unter Verwendung von SDS-Polyacrylamid-Gelelektrophorese, und der zweite Antikörper ein Antikörper ist, der eine Antigen-Antikörper-Reaktion durch Western-Blotting mit einem vollständigen Influenza-A-Virus-Kernprotein auslöst, das abgetrennt wird unter Verwendung von SDS-Polyacrylamid-Gelelektrophorese.

5. Verfahren zum Nachweisen von Influenza-A-Virus durch Immunchromatographie nach Anspruch 4,
wobei der erste Antikörper ein oder mehr monoklonale Antikörper ist.

6. Verfahren zum Nachweisen von Influenza-A-Virus durch Immunchromatographie nach Anspruch 4 oder 5,
wobei der erste Antikörper ein Antikörper ist, der durch Immunisieren eines vollständigen Kernproteins von Influenza-A-Virus des Subtyps H1N1 erhalten wird.

## Revendications

1. Trousse de détection du virus de la grippe A par immunochromatographie, comprenant :
un milieu chromatographique dans lequel est immobilisé un premier anticorps causant une réaction antigène-anticorps avec une protéine nucléaire du virus de la grippe A mais ne causant pas de réaction antigène-anticorps avec une protéine nucléaire du virus de la grippe B, et
un réactif de marquage dans lequel un deuxième anticorps causant une réaction antigène-anticorps avec une protéine nucléaire du virus de la grippe A mais ne causant pas de réaction antigène-anticorps avec une protéine nucléaire du virus de la grippe B est conjugué à une substance de marquage ;
le premier anticorps étant un anticorps ne causant pas de réaction antigène-anticorps avec une protéine nucléaire complète du virus de la grippe A séparée par électrophorèse sur gel de polyacrylamide en présence de SDS, dans le cadre d'un immunobuvardage, et le deuxième anticorps étant un anticorps causant une réaction anticorps-antigène, dans le cadre d'un immunobuvardage, avec une protéine nucléaire complète du virus de la grippe A séparée par électrophorèse sur gel de polyacrylamide en présence de SDS.

2. Trousse de détection du virus de la grippe A par immunochromatographie selon la revendication 1,
dans laquelle le premier anticorps consiste en au moins un anticorps monoclonal.

3. Trousse de détection du virus de la grippe A par immunochromatographie selon la revendication 1 ou 2,
dans laquelle le premier anticorps est un anticorps obtenu par immunisation d'une protéine nucléaire complète du virus de la grippe A de sous-type H1N1.

4. Méthode de détection du virus de la grippe A par immunochromatographie, mettant en oeuvre :
un milieu chromatographique dans lequel est immobilisé un premier anticorps causant une réaction antigène-anticorps avec une protéine nucléaire du virus de la grippe A mais ne causant pas de réaction antigène-anticorps avec une protéine nucléaire du virus de la grippe B, et
un réactif de marquage dans lequel un deuxième anticorps causant une réaction antigène-anticorps avec une protéine nucléaire du virus de la grippe A mais ne causant pas de réaction antigène-anticorps avec une protéine nucléaire du virus de la grippe B est conjugué à une substance de marquage ;
le premier anticorps étant un anticorps ne causant pas de réaction antigène-anticorps, dans le cadre d'un immunobuvardage, avec une protéine nucléaire complète du virus de la grippe A séparée par électrophorèse sur gel de polyacrylamide en présence de SDS, et le deuxième anticorps étant un anticorps causant une réaction antigène-anticorps, dans le cadre d'un immunobuvardage, avec une protéine nucléaire complète du virus de la grippe A séparée par électrophorèse sur gel de polyacrylamide en présence de SDS.

5. Méthode de détection du virus de la grippe A par immunochromatograhie selon la revendication 4,
dans laquelle le premier anticorps consiste en au moins un anticorps monoclonal.

6. Méthode de détection du virus de la grippe A par immunochromatograhie selon la revendication 4 ou 5,
dans laquelle le premier anticorps est un anticorps obtenu par immunisation d'une protéine nucléaire complète du virus de la grippe A de sous-type H1N1.
